(19) Europäisches Patentamt
European Patent Office
Office européen des brevets

(11) Veröffentlichungsnummer: **0 017 946**
**B1**

(12) # EUROPÄISCHE PATENTSCHRIFT

(45) Veröffentlichungstag der Patentschrift: **14.04.82**

(21) Anmeldenummer: **80101987.8**

(22) Anmeldetag: **14.04.80**

(51) Int. Cl.³: **C 07 C 103/50**, C 07 D 295/14, C 07 D 209/08, C 07 D 215/08, C 07 D 211/14, C 07 D 211/22, C 07 D 211/42, C 07 D 211/70, A 01 N 37/44, A 01 N 43/34, A 01 N 43/84

(54) **Salze von Alpha-Aminoacetaniliden, Verfahren zu ihrer Herstellung und ihre Anwendung als Pflanzenwachstumsregulatoren.**

(30) Priorität: **14.04.79 DE 2915250**

(43) Veröffentlichungstag der Anmeldung: **29.10.80 Patentblatt 80/22**

(45) Bekanntmachung des Hinweises auf die Patenterteilung: **14.04.82 Patentblatt 82/15**

(84) Benannte Vertragsstaaten: **AT BE CH DE FR GB IT LI LU NL SE**

(56) Entgegenhaltungen:
**FR-A- 2 336 079**
**US-A- 3 014 046**
**CHEMICAL ABSTRACTS, Band 61, Nr. 5, 31. August 1964, Zusammenfassung Nr. 5617c COLUMBUS OHIO (US).**

(73) Patentinhaber: **BASF Aktiengesellschaft, Carl-Bosch-Strasse 38, D-6700 Ludwigshafen (DE)**

(72) Erfinder: **Buschmann, Ernst, Dr., An der Froschlache 7, D-6700 Ludwigshafen (DE)**
Erfinder: **Schirmer, Ulrich, Dr., Berghalde 79, D-6900 Heidelberg 1 (DE)**
Erfinder: **Zeeh, Bernd, Dr., Thorwaldsenstrasse 5, D-6700 Ludwigshafen (DE)**
Erfinder: **Jung, Johann, Dr., Hardenburgstrasse 19, D-6703 Limburgerhof (DE)**
Erfinder: **Sauter, Hubert, Dr., Neckarpromenade 20, D-6800 Mannheim 1 (DE)**

**EP 0 017 946 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Salze von α-Aminoacetaniliden, Verfahren zu ihrer Herstellung und ihre Anwendung als Pflanzenwachstumsregulatoren

Die vorliegende Erfindung betrifft neue quartäre Ammoniumsalze von α-Aminoacetaniliden sekundärer Aniline mit guter biologischer Wirkung, Mittel zur Regulierung des Pflanzenwachstums, die diese Ammoniumsalze enthalten, und die Verwendung von α-Aminoacetaniliden sekundärer Aniline zur Herstellung der neuen Salze.

Es ist bekannt, dass quartäre Ammoniumsalze von α-Aminoacetaniliden primärer Aniline der Formel III

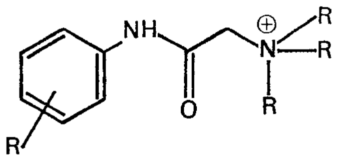

$X^{\ominus}$ III

das Pflanzenwachstum beeinflussen (DE-OS 2657728).

Ferner ist es bekannt, dass in photosynthesehemmenden Arylharnstoffen, Aniliden und N-Arylcarbamaten dem Strukturelement ArNHCOR essentielle Bedeutung zukommt. Der Übergang von Derivaten primärer Aniline zu Derivaten sekundärer Aniline (Strukturelement ArNAlkylCOR) bringt immer eine Verminderung bzw. eine Blockierung der biologischen Aktivität mit sich [A. Barth u. H.-J. Michel, Pharmazie 24, 11-23 (1969), N.D. Camper u. D.E. Moreland, Biochim. Biophys. Acta 94, 383 (1965), N.E. Good, Plant Physiology 36, 788 (1961)].

Aus der US-Patentschrift 3014046 sind quaternäre Ammoniumjodide bekannt, die als Entlaubungsmittel für Pflanzen wirken. Hierdurch werden die erfindungsgemässen Mittel zur Regulierung des Pflanzenwachstums nicht nahegelegt, weil Entlaubung von Pflanzen (Schädigung) und Regulierung des Pflanzenwachstums ohne Schädigung der Pflanzen sich gegenseitig ausschliessen. In der Veröffentlichung werden die Einzelwirkstoffe N-[2-(2,6-Dimethylanilino)-2-oxo]-ethyl-N-triethylammonium-bromid und N-[2-(2,6-Dimethylanilino)-2-oxo]-ethyl-N-triethylammonium-chlorid genannt.

Es wurde nun überraschenderweise gefunden, dass die neuen quartären Ammoniumsalze von α-Aminoacetaniliden sekundärer Aniline der Formel I

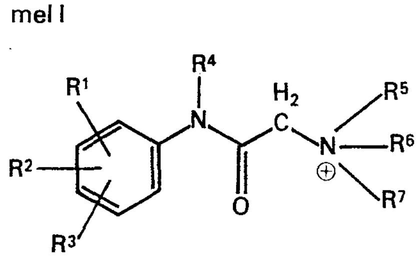

$X^{\ominus}$ I,

in der $R^1$, $R^2$, $R^3$ unabhängig voneinander Wasserstoff, einen Alkylrest (z.B. Methyl, Ethyl, Isopropyl), einen Cycloalkyl-, Aralkyl-, Alkoxyrest (z.B. Methoxy), wobei zwei benachbarte Reste einen heterocyclischen Ring mit 1 oder 2 Sauerstoffatomen bilden können, Halogen (z.B. Br, Cl, F), Halogenalkyl (z.B. $CF_3$, $CF_2Cl$), $NO_2$, Acyl, Alkoxyalkyl, Halogenalkoxy, Alkylthio, Halogenalkylthio, Aryl, Thiocyanato, Cyano, NHCOR′, NHCONR′R″, COOR′, CONR′R″, $SO_2R'$ oder $SO_2NR'R''$ bedeuten, wobei R′ und R″ unabhängig voneinander Wasserstoff, Alkyl, Cycloalkyl, Aryl und Aralkyl bedeuten, $R^4$ einen Alkylrest (z.B. Methyl, Ethyl, Propyl, Butyl, Isobutyl), Aralkylrest (z.B. Benzyl), einen Alkenylrest (z.B. Allyl, Crotyl), einen Alkinylrest (z.B. Propargyl), wobei der Alkylrest mit der Orthoposition des aromatischen Ringes verknüpft sein kann, $R^5$, $R^6$, $R^7$ unabhängig voneinander gleiche oder verschiedene Reste aus der Gruppe der Alkyl-, Alkenyl-, Alkinyl-, Cyanoalkyl-, Halogenalkyl-, Hydroxyalkyl- oder Alkoxyalkylreste bedeuten, wobei zwei Alkylreste mit dem Stickstoffatom, dessen Substituenten sie sind, auch einen heterocyclischen Ring (z.B. Pyrrolidin, Piperidin, Hexamethylenimin) bilden können, der eine Doppelbindung (z.B. 1,2,5,6-Tetrahydropyrimidin) enthalten kann, in dem ein Methylenrest durch ein Sauerstoffatom oder ein Schwefelatom (z.B. Morpholin, Thiomorpholin) ersetzt sein kann und der durch Hydroxy-, Alkoxy-, Hydroxymethylen- und Alkylgruppen substituiert sein kann und $X^{\ominus}$ das Anion einer Säure der Formel HBr, HCl, $HNO_3$, $CH_3OSO_3H$ oder $CF_3SO_3H$ ausser N-[-(2,6-Dimethylanilino)-2-oxo]-ethyl-N-triethylammonium-bromid und N-[2-(2,6-Dimethylanilino)-2-oxo]-ethyl-N-triethylammonium-chlorid bedeutet, das Pflanzenwachstum beeinflussen und in der wachstumsregulierenden Wirkung den in der DE-OS 2657728 beschriebenen Derivaten primärer Aniline der Formel III überraschend überlegen sind.

$R^1$, $R^2$, $R^3$ bedeuten unabhängig voneinander beispielsweise Wasserstoff, Fluor, Chlor, Brom, Jod, Trifluormethyl, Trichlormethyl, Difluorchlormethyl, Methoxy, Ethoxy, Propoxy, $NO_2$, Methyl, Ethyl, Propyl, Isopropyl, t-Butyl, Benzyl, Cyclopentyl, Cyclohexyl, Cycloheptyl, Acetyl, Propionyl, Butyryl, Isobutyryl, Benzoyl, Methoxymethyl, Ethoxymethyl, Trifluormethoxy, Thiomethyl, Thioethyl, Thioisobutyl, Phenyl, CN, $NHCOCH_3$, $NHCONHCH_3$, $NHCON(CH_3)_2$, $CO_2Me$, $CO_2Et$.

$R^4$ bedeutet beispielsweise Methyl, Ethyl, Propyl, Isopropyl, Butyl, Isobutyl, Allyl, Crotyl, Prenyl, Isoprenyl, Propargyl, 2-Butinyl, Benzyl.

$R^5$ bis $R^7$ bedeuten unabhängig voneinander beispielsweise Methyl, Ethyl, Propyl, Butyl, Isobutyl, Allyl, Crotyl, Prenyl, Isoprenyl, Propargyl, Butinyl, $CH_2CN$, 2-Chlorethyl, 2-Bromethyl, 2-Hydroxyethyl, 2-Methoxyethyl.

Zwei der Reste $R^5$ bis $R^7$ können mit dem Stickstoffatom, dessen Substituenten sie sind, auch einen heterocyclischen Ring bilden. Solche heterocyclischen Ringe sind beispielsweise Pyrrolidin, Piperidin, 3-Methylpiperidin, 4-Methylpiperidin, 3,5-Dimethylpiperidin, 3,3-Dimethylpiperidin, Hexamethylenimin, 3,5,5-Trimethylhexamethylenimin, Morpholin, 2,6-Dimethylmorpholin,

Thiomorpholin, 2,6-Dimethylthiomorpholin, 3-Hydroxymethylpiperidin, 3-Methoxymethylpiperidin, 3-Hydroxypiperidin, 4-Hydroxypiperidin, 3-Methoxypiperidin, 4-Methoxypiperidin.

Die erfindungsgemässen Wirkstoffe können als Wachstumsregulatoren verwendet werden.

Für die Wirkungsweise von Pflanzenwachstumsregulatoren gilt, dass sie mehrere verschiedenartige Wirkungen auf Pflanzen ausüben können. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwachstumregulierende Stoffe können z.B. zur Hemmung des vegetativen Pflanzenwachstums eingesetzt werden. Eine derartige Wuchshemmung ist u.a. bei Gräsern von wirtschaftlichem Interesse, denn durch eine Dämpfung des Graswachstums kann z.B. die Häufigkeit des Grasmähens in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung, so dass ein Mehrertrag bezogen auf die Bodenfläche erzielt werden kann.

Ein weiterer Mechanismus der Ertragssteigerung mit Pflanzenwachstumsregulatoren beruht darauf, dass die Nährstoffe in stärkerem Masse der Blüten- und Fruchtbildung zugute kommen, während das vegetative Wachstum eingeschränkt wird.

Mit Pflanzenwachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel erreicht werden, ohne dass sich Änderungen des vegetativen Wachstums bemerkbar machen. Wachstumsregulatoren können ferner eine Veränderung der Zusammensetzung der Pflanzen bewirken, um so eine bessere Qualität der Ernteprodukte herbeizuführen. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Durch Einsatz von Wachstumsregulatoren lässt sich der vorzeitige Fruchtfall verhindern. Es ist jedoch auch möglich, den Fruchtfall – z.B. bei Obst – im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Hilfe von Wachstumsregulatoren auch eine zeitliche Konzentrierung der Reife erzielt werden. Damit werden die Voraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch Anwendung von Wachstumsregulatoren kann auch die Samen- oder Knospenruhe der Pflanzen, also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden.

Wachstumsregulatoren können auch eine Halophilie bei Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Die neuen α-Ammoniumacetanilide der Formel I können nach folgendem Schema hergestellt werden:

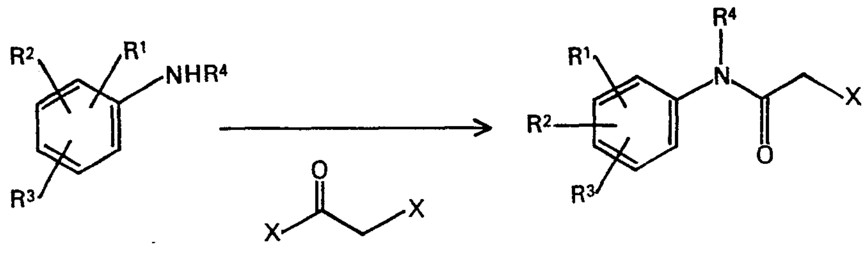

V VI VII

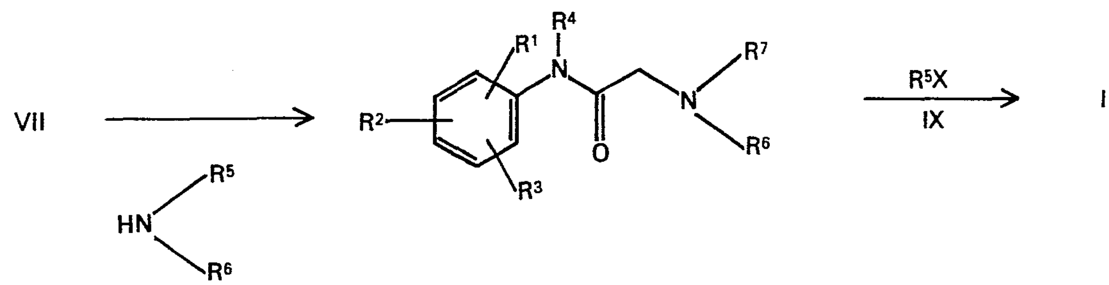


VII VIII II I

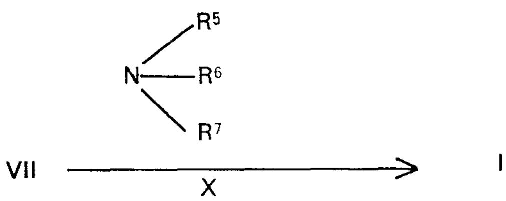

VII X I

X = Chlor- oder Bromatom

Ein sekundäres Anilin der Formel V wird mit einem α Halogencarbonsäurehalogenid der Formel VI zum α-Halogencarbonsäureanilid VII umgesetzt (siehe z.B. Houben-Weyl, Band 11/2, S. 35). Die α-Halogencarbonsäureanilide VII werden mit sekundären Aminen VIII zu α-Aminoacetaniliden II umgesetzt und anschliessend mit den Alkylierungsmitteln IX zu den erfindungsgemässen Verbindungen I quaternisiert. Alternativ kann man die Ammoniumsalze I auch direkt aus den α-Halogencarbonsäureaniliden VII und den tertiären Aminen X herstellen.

Aniline der Formel V sind z.B. N-Methylanilin, N-Ethylanilin, N-Propylanilin, 4-Chlor-N-methylanilin, 2,4-Chlor-N-methylanilin, 4-Chlor-2,N-dimethylanilin, N-Allyl-4-chlor-2-methylanilin, 4-Chlor-N-ethyl-2-methylanilin, 4-Chlor-2-methyl-N-propylanilin, 4-Chlor-2-methyl-N-propargylanilin, N-Methyl-3-trifluormethylanilin, 3-Chlor-4-fluor-N-methylanilin, 3,4-Difluor-N-methylanilin, 3-Methoxy-N-methylanilin, N-Methyl-3-nitroanilin, 3-Ethyl-5,N-dimethylanilin, 3-Isopropyl-N-methylanilin, 4-Fluor-3,N-dimethylanilin, 2,4-Difluor-N-methylanilin, 4-Fluor-3,N-dimethylanilin, 3-Chlor-N-methylanilin.

Halogencarbonsäurehalogenide der Formel VI sind beispielsweise α-Chloracetylchlorid und α-Bromacetylbromid.

Sekundäre Amine der Formel VIII sind z.B. Pyrrolidin, Piperidin, Hexamethylenimin, Morpholin, 3,5-Dimethylmorpholin, Thiamorpholin, Diethylamin, Dipropylamin, Diallylamin, 1,2,3,6-Tetrahydropyrimidin, 3-Hydroxypiperidin, 3-Hydroxymethylenpiperidin, 3,5-Dimethylpiperidin, 3-Methylpiperidin, 4-Methylpiperidin, 4-Hydroxypiperidin, 2,6-Dimethylmorpholin, 2,6-Dimethylthiomorpholin.

Quarternisierungsmittel der Formel IX sind beispielsweise: Methylbromid, Bromethan, Brompropan, Brombutan, 3-Methylbrombutan, Allylbromid, Crotylbromid, Propargylbromid.

Tertiäre Amine der Formel X sind beispielsweise: N-Methylpyrrolidin, N-Ethylpyrrolidin, N-Propylpyrrolidin, N-Methylpiperidin, N-Ethylpiperidin, N-Propylpiperidin, N-Methylmorpholin.

Die Umsetzung einer Verbindung der Formel IX erfolgt beispielsweise bei einer Temperatur von 10 bis 150 °C in Anwesenheit oder Abwesenheit eines Lösungsmittels z.B. Ethanol, Methanol, Chloroform, Methylenchlorid, Acetonitril, Dimethylformamid oder Essigester bei Normaldruck oder erhöhtem Druck.

Die Ausgangsstoffe werden bevorzugt mit einem Überschuss von 10 bis 100% an Verbindung IX über die stöchiometrische Menge hinaus, bezogen auf Verbindung II, umgesetzt.

Die Umsetzung einer Verbindung der Formel VII mit einer Verbindung der Formel X erfolgt beispielsweise bei einer Temperatur von 10 bis 150 °C in Anwesenheit oder Abwesenheit eines Lösungsmittels, z.B. Ethanol, Methanol, Chloroform, Methylenchlorid, Acetonitril, Dimethylformamid oder Essigester bei Normaldruck oder erhöhtem Druck.

Die Ausgangsprodukte werden in stöchiometrischen Mengen oder mit einem Überschuss von 10 bis 100% an Verbindung X über die stöchiometrische Menge hinaus, bezogen auf Verbindung VII, umgesetzt.

Die nachstehenden Vorschriften und Beispiele erläutern die Herstellung der erfindungsgemässen Wirkstoffe der Formel I und ihrer Vorprodukte.

Vorschrift A für die Synthese der Halogencarbonsäureanilide der Formel VII

N-Chloracetyl-N-methyl-4-chlor-2-methyl-anilin (A0)

Zu einer Lösung von 60 g N-Methyl-4-chlor-2-methyl-anilin in 150 ml Aceton werden 44 g Natriumacetat in 200 ml Wasser zugegeben. Anschliessend werden 56 g Chloracetylchlorid innerhalb 3 Stunden zugetropft. Die Temperatur wird dabei unter 35 °C gehalten. Nach 2 Stunden Rühren bei Raumtemperatur werden 200 ml Eiswasser zugegeben. Man kühlt auf 5 bis 10 °C ab, saugt ab, wäscht mit Wasser und trocknet bei 60 °C im Vakuum. Ausbeute 85 g. Schmp. 85–87 °C.

Vorschrift B für die Synthese der α-Aminoacetanilide der Formel II

N-Pyrrolidinoacetyl-N-methyl-4-chlor-2-methyl-anilin (A1)

Zu einer Lösung von 46,4 g der Verbindung A0 in 300 ml Äthanol werden 42,6 g Pyrrolidin zugetropft. Die Reaktionstemperatur wird unter 40 °C gehalten. Man rührt 3 Stunden bei Raumtemperatur, dampft Äthanol ab, löst in Äther/Wasser, extrahiert die wässrige Phase noch mehrmals mit Äther, wäscht die vereinigten organischen Phasen mit Wasser, trocknet über $Na_2SO_4$ und dampft Äther ab. Das Produkt kristallisiert nach Verreiben mit n-Pentan. Ausbeute 46 g. Schmp. 59 °C.

In entsprechender Weise können die in den folgenden zwei Tabellen aufgeführten α-Aminoacetanilide sekundärer Amine hergestellt werden.

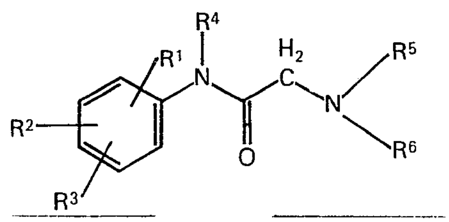

| Nr. | $R^1$ | $R^2$ | $R^3$ | $R^4$ | $R^5/R^6$ | Schmp. |
|---|---|---|---|---|---|---|
| A1 | 2-$CH_3$ | 4-Cl | H | $CH_3$ | $(CH_2)_4$ | 59°C |
| A2 | 2-$CH_3$ | 4-Cl | H | $CH_3$ | $(CH_2)_5$ | Öl |
| A3 | 3-$CF_3$ | H | H | $CH_3$ | $(CH_2)_4$ | |
| A4 | 4-F | 3-Cl | H | $CH_3$ | $(CH_2)_4$ | |
| A5 | 3-$CH_3$ | H | H | $CH_3$ | $(CH_2)_5$ | |
| A6 | 2-F | 4-F | H | $CH_3$ | $(CH_2)_4$ | |
| A7 | 3-$OCH_3$ | H | H | $CH_3$ | $(CH_2)_4$ | |
| A8 | 3-$NO_2$ | H | H | $CH_3$ | $(CH_2)_4$ | |
| A9 | 3-$C_2H_5$ | 5-$CH_3$ | H | $CH_3$ | $(CH_2)_4$ | |
| A10 | 3-i-Prop | H | H | $CH_3$ | $(CH_2)_4$ | |
| A11 | 3-$CH_3$ | 4-F | H | $CH_3$ | $(CH_2)_4$ | |
| A12 | 4-Cl | H | H | $CH_3$ | $(CH_2)_4$ | |
| A13 | 3-$C_2H_5$ | 5-$CH_3$ | H | $CH_3$ | $(CH_2)_5$ | |
| A14 | 3-$C_2H_5$ | 5-$CH_3$ | H | $CH_3$ | $(CH_2)_6$ | |
| A15 | 3-$OCH_3$ | H | H | $CH_3$ | $(CH_2)_5$ | |
| A16 | 2-F | 4-F | H | $CH_3$ | $(CH_2)_5$ | |
| A17 | 4-Cl | H | H | $CH_3$ | $(CH_2)_5$ | |
| A18 | 3-$NO_2$ | H | H | $CH_3$ | $(CH_2)_5$ | |
| A19 | 3-$CH_3$ | 4-F | H | $CH_3$ | $(CH_2)_5$ | |

| Nr. | $R^1$ | $R^2$ | $R^3$ | $R^4$ | $R^5/R^6$ | Schmp. |
|---|---|---|---|---|---|---|
| A20 | 3-i-Prop | H | H | $CH_3$ | $(CH_2)_5$ | |
| A21 | 2-$CH_3$ | 4-Cl | H | $CH_3$ | $(CH_2)_6$ | |
| A22 | 2-$CH_3$ | 4-Cl | H | $CH_3$ | $CH_2CHCH_3(CH_2)_3$ | |
| A23 | 2-$CH_3$ | 4-Cl | H | $CH_3$ | Propyl/Propyl | |
| A24 | 4-F | 3-$CH_3$ | H | $CH_3$ | $(CH_2)_6$ | |
| A25 | 2-$CH_3$ | 4-Cl | H | Allyl | $(CH_2)_4$ | |
| A26 | 2-$CH_3$ | 4-Cl | H | $CH_3$ | Ethyl/Ethyl | |
| A27 | 2-$CH_3$ | 4-Cl | H | $CH_3$ | $[(CH_2)_2]_2O$ | |
| A28 | 2-$CH_3$ | 4-Cl | H | $CH_3$ | $(CH_2CHCH_3)_2O$ | |
| A29 | 4-Br | H | H | $CH_3$ | $(CH_2)_4$ | |
| A30 | H | H | H | $CH_3$ | $(CH_2)_4$ | |
| A31 | 2-Cl | 4-Cl | 6-Cl | $CH_3$ | $(CH_2)_4$ | |
| A32 | 2-Cl | 3-Cl | 4-Cl | $CH_3$ | $(CH_2)_4$ | |
| A33 | 2-$CH_3$ | 4-Cl | 5-Cl | $CH_3$ | $(CH_2)_4$ | |
| A34 | 2-$CH_3$ | 4-Cl | H | $C_2H_5$ | $(CH_2)_4$ | |
| A35 | 2-$CH_3$ | 4-Cl | H | n-Propyl | $(CH_2)_4$ | |
| A36 | 2-$CH_3$ | 4-Cl | H | Propargyl | $(CH_2)_4$ | |
| A37 | 2-$CH_3$ | 4-Cl | H | Isopropyl | $(CH_2)_4$ | |

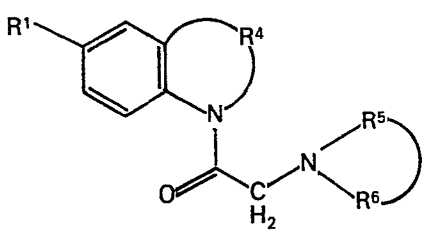

| Nr. | $R^1$ | $R^5/R^6$ | $R^4$ |
|---|---|---|---|
| A38 | H | $(CH_2)_4$ | $(CH_2)_2$ |
| A39 | H | $(CH_2)_5$ | $(CH_2)_2$ |
| A40 | H | $(CH_2)_4$ | $(CH_2)_3$ |
| A41 | H | $(CH_2)_5$ | $(CH_2)_3$ |
| A42 | Cl | $(CH_2)_4$ | $(CH_2)_2$ |
| A43 | Cl | $(CH_2)_4$ | $(CH_2)_3$ |
| A44 | Br | $(CH_2)_4$ | $(CH_2)_2$ |
| A45 | Br | $(CH_2)_4$ | $(CH_2)_3$ |

Beispiel 1

Herstellung von N-[2-(4-Chlor-2-methyl-N-methyl-anilino)-2-oxo]-ethyl-N-allyl-pyrrolidiniumbromid

Eine Lösung von 46 g der Verbindung A1, 42 g 1-Brompropen-2 in 200 ml Essigester wird 4 Stunden zum Rückfluss erwärmt. Nach dem Abkühlen wird das ausgefallene Produkt abgesaugt, mit Essigester gewaschen und getrocknet.

Ausbeute 62 g, Schmp. 173–175 °C.

Beispiel 2

N-[2-(4-Chlor-2-methyl-N-methyl-anilino)-2-oxo]-ethyl-N-ethyl-pyrrolidiniumbromid

Eine Lösung von 20 g der Verbindung A1, 15,9 g Ethylbromid in 200 ml Acetonitril wird 14 Stunden bei Raumtemperatur gerührt und anschliessend eingeengt. Der kristalline Rückstand wird mit Ether verrieben, abgesaugt und getrocknet.

Ausbeute 25 g, Schmp. 179–181 °C.

Beispiel 3

N-[2-(4-Chlor-2-methyl-N-methyl-anilino)-2-oxo]-ethyl-N-methyl-piperidiniumchlorid

Zu einer Lösung von 46,4 g der Verbindung A0 in 150 ml Acetonitril werden 40 g N-Methylpiperidin zugetropft. Die Reaktionstemperatur wird dabei unter 25 °C gehalten. Nach 14 Stunden wird das ausgefallene Produkt mit Ether gewaschen und getrocknet.

Ausbeute 52 g, Schmp. höher als 230 °C.

Beispiel 4

N-[2-(4-Chlor-2-methyl-N-methyl-anilino)-2-oxo]-ethyl-N-methyl-pyrrolidiniumbromid

Eine Lösung von 21 g Brommethan, 30 g der Verbindung A1 in 300 ml Acetonitril wird 14 Stunden bei Raumtemperatur gerührt. Das auskristallisierte Produkt wird abgesaugt, mit Ether gewaschen und getrocknet.

Ausbeute 37 g, Schmp. 157 °C.

In entsprechender Weise können die in den folgenden Tabellen aufgeführten erfindungsgemässen Verbindungen hergestellt werden.

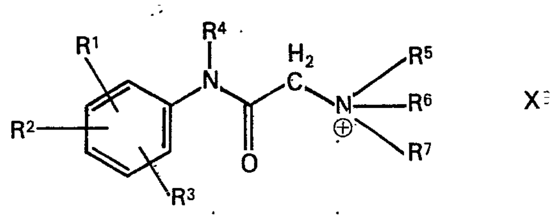

| Nr. | $R^1$ | $R^2$ | $R^3$ | $R^4$ | $R^5$ | $R^6/R^7$ | X | Schmp. °C Z = Zersetzung |
|---|---|---|---|---|---|---|---|---|
| 1 | 2-$CH_3$ | 4-Cl | H | $CH_3$ | Allyl | $(CH_2)_4$ | Br | 173–175 Z |
| 2 | 2-$CH_3$ | 4-Cl | H | $CH_3$ | Allyl | $(CH_2)_5$ | Br | Harz |
| 3 | 3-$CF_3$ | H | H | $CH_3$ | Allyl | $(CH_2)_4$ | Br | Harz |
| 4 | 4-F | 3-Cl | H | $CH_3$ | Allyl | $(CH_2)_4$ | Br | 124–126 |
| 5 | 3-$CF_3$ | H | H | $CH_3$ | Allyl | $(CH_2)_5$ | Br | Harz |
| 6 | 2-F | 4-F | H | $CH_3$ | Allyl | $(CH_2)_4$ | Br | 172 |
| 7 | 3-$OCH_3$ | H | H | $CH_3$ | Allyl | $(CH_2)_4$ | Br | 145–147 Z |
| 8 | 3-$NO_2$ | H | H | $CH_3$ | Allyl | $(CH_2)_4$ | Br | 128–130 |
| 9 | 3-$C_2H_5$ | 5-$CH_3$ | H | $CH_3$ | Allyl | $(CH_2)_4$ | Br | 135–136 |
| 10 | 3-i-Prop | H | H | $CH_3$ | Allyl | $(CH_2)_4$ | Br | Harz |
| 11 | 3-$CH_3$ | 4-F | H | $CH_3$ | Allyl | $(CH_2)_4$ | Br | Harz |
| 12 | 4-Cl | H | H | $CH_3$ | Allyl | $(CH_2)_4$ | Br | 100–103 |
| 13 | 3-$C_2H_5$ | 5-$CH_3$ | H | $CH_3$ | Allyl | $(CH_2)_5$ | Br | 123–125 |
| 14 | 3-$C_2H_5$ | 5-$CH_3$ | H | $CH_3$ | Allyl | $(CH_2)_6$ | Br | 90–92 |
| 15 | 3-$OCH_3$ | H | H | $CH_3$ | Allyl | $(CH_2)_5$ | Br | 109–110 |
| 16 | 2-F | 4-F | H | $CH_3$ | Allyl | $(CH_2)_5$ | Br | 182 |
| 17 | 4-Cl | H | H | $CH_3$ | Allyl | $(CH_2)_5$ | Br | 144–146 |
| 18 | 3-$NO_2$ | H | H | $CH_3$ | Allyl | $(CH_2)_5$ | Br | 170–172 Z |
| 19 | 3-$CH_3$ | 4-F | H | $CH_3$ | Allyl | $(CH_2)_5$ | Br | 96–101 |
| 20 | 3-i-Prop | H | H | $CH_3$ | Allyl | $(CH_2)_5$ | Br | Harz |
| 21 | 2-$CH_3$ | 4-Cl | H | $CH_3$ | Allyl | $(CH_2)_6$ | Br | 91–98 |
| 22 | 2-$CH_3$ | 4-Cl | H | $CH_3$ | Crotyl | $(CH_2)_4$ | Br | 171–175 Z |
| 23 | 2-$CH_3$ | 4-Cl | H | $CH_3$ | Allyl | $CH_2CHCH_3(CH_2)_3$ | Br | 84–85 |
| 24 | 2-$CH_3$ | 4-Cl | H | $CH_3$ | Allyl | Propyl/Propyl | Br | |
| 25 | 2-$CH_3$ | 4-Cl | H | $CH_3$ | Propargyl | $(CH_2)_4$ | Br | 175–178 Z |
| 26 | 2-$CH_3$ | 4-Cl | H | $CH_3$ | $CH_3$ | $(CH_2)_4$ | Br | 157 Z |
| 27 | 4-F | 3-$CH_3$ | H | $CH_3$ | Allyl | $(CH_2)_6$ | Br | 180 Z |
| 28 | 2-$CH_3$ | 4-Cl | H | Allyl | Allyl | $(CH_2)_4$ | Br | Harz |
| 29 | 2-$CH_3$ | 4-Cl | H | $CH_3$ | Ethyl | $(CH_2)_4$ | Br | 179–181 |
| 30 | 2-$CH_3$ | 4-Cl | H | $CH_3$ | Propyl | $(CH_2)_4$ | Br | 208–210 |
| 31 | 2-$CH_3$ | 4-Cl | H | $CH_3$ | Butyl | $(CH_2)_4$ | Br | 170–172 |
| 32 | 2-$CH_3$ | 4-Cl | H | $CH_3$ | Ethyl | Ethyl/Ethyl | Cl | |
| 33 | 2-$CH_3$ | 4-Cl | H | $CH_3$ | $CH_3$ | $[(CH_2)_2]_2O$ | Cl | 210–211 Z |
| 34 | 2-$CH_3$ | 4-Cl | H | $CH_3$ | Allyl | $[(CH_2)_2]_2O$ | Br | 166–168 Z |
| 35 | 2-$CH_3$ | 4-Cl | H | $CH_3$ | Allyl | $(CH_2CHCH_3)_2O$ | Br | 168–169 Z |
| 36 | 2-$CH_3$ | 4-Cl | H | $CH_3$ | Allyl | $CH_2CHOH(CH_2)_3$ | Br | 81–83 |
| 37 | 2-$CH_3$ | 4-Cl | H | $CH_3$ | Allyl | $CH_2CH{=}CH(CH_2)_2$ | Br | 148–149 Z |
| 38 | 2-$CH_3$ | 4-Cl | H | $CH_3$ | Allyl | $CH_2CHCH_2OH(CH_2)_3$ | Br | 86–88 |
| 39 | 2-$CH_3$ | 4-Cl | H | $CH_2H_5$ | Allyl | $(CH_2)_4$ | Br | 50 |
| 40 | 2-$CH_3$ | 4-Cl | H | n-$C_3H_7$ | Allyl | $(CH_2)_4$ | Br | |
| 41 | 2-$CH_3$ | 4-Cl | H | i-$C_3H_7$ | Alyl | $(CH_2)_4$ | Br | |
| 42 | 2-$CH_3$ | 4-Cl | H | Propar-gyl | Allyl | $(CH_2)_4$ | Br | Harz |
| 43 | 2-$CH_3$ | 4-Cl | H | $CH_3$ | $(CH_2)_2CH(CH_3)_2$ | $(CH_2)_4$ | Br | 168–169 Z |
| 44 | 2-$CH_3$ | 4-Cl | H | $CH_3$ | $CH_3$ | $(CH_2)_5$ | Cl | 230 |
| 45 | 2-$CH_3$ | 4-Cl | H | $CH_3$ | Allyl | $(CH_2)_4$ | $CH_3OSO_3^{\ominus}$ | |
| 46 | 2-$CH_3$ | 4-Cl | H | $CH_3$ | $CH_3$ | $(CH_2)_4$ | $CF_3SO_3^{\ominus}$ | |
| 47 | 2-Cl | 4-Cl | 6-Cl | $CH_3$ | Allyl | $(CH_2)_4$ | Br | |
| 48 | 2-Cl | 3-Cl | 4-Cl | $CH_3$ | Allyl | $(CH_2)_4$ | Br | |
| 49 | 2-$CH_3$ | 4-Cl | 5-Cl | $CH_3$ | Allyl | $(CH_2)_4$ | Br | |
| 50 | 2-$CH_3$ | 4-Cl | H | $C_2H_5$ | $CH_3$ | $(CH_2)_4$ | Br | 60 |
| 51 | 2-$CH_3$ | 4-Cl | H | $C_2H_5$ | $CH_3$ | $(CH_2)_5$ | Br | 88–89 |
| 52 | 2-$CH_3$ | 4-Cl | H | $C_2H_5$ | Allyl | $(CH_2)_5$ | Br | 88–90 |

| Nr. | $R^1$ | $R^2$ | $R^3$ | $R^4$ | $R^5$ | $R^6/R^7$ | X | Schmp. °C Z = Zersetzung |
|---|---|---|---|---|---|---|---|---|
| 53 | 2-$CH_3$ | 4-Cl | H | $CH_2C_6H_5$ | Allyl | $(CH_2)_4$ | Br | |
| 54 | H | H | H | $CH_3$ | Allyl | $(CH_2)_4$ | Br | 136 Z |
| 55 | 3-Cl | H | H | $CH_3$ | Allyl | $(CH_2)_4$ | Br | 134 Z |
| 56 | 3-Cl | H | H | $CH_3$ | $CH_3$ | $(CH_2)_4$ | Br | 123 Z |
| 57 | H | H | H | $CH_3$ | $CH_3$ | $(CH_2)_4$ | Br | 130 Z |
| 58 | H | H | H | $CH_3$ | $C_2H_5$ | $(CH_2)_4$ | Br | 57 |
| 59 | 3-Cl | H | H | $CH_3$ | $C_2H_5$ | $(CH_2)_4$ | Br | 129 Z |

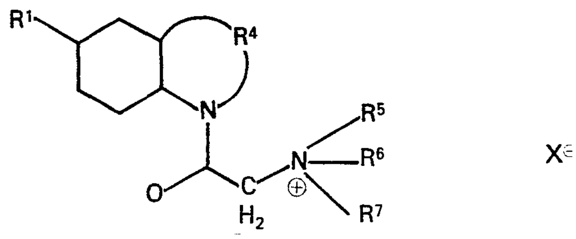

| Nr. | $R^1$ | $R^5/R^6$ | $R^7$ | $R^4$ | X | Schmp. °C |
|---|---|---|---|---|---|---|
| 60 | H | $(CH_2)_4$ | Allyl | $(CH_2)_2$ | Br | 178 Z |
| 61 | H | $(CH_2)_4$ | Allyl | $(CH_2)_3$ | Br | 160 Z |
| 62 | H | $(CH_2)_5$ | Allyl | $(CH_2)_3$ | Br | Harz |
| 63 | H | $(CH_2)_5$ | Allyl | $(CH_2)_2$ | Br | |
| 64 | Cl | $(CH_2)_4$ | Allyl | $(CH_2)_2$ | Br | |
| 65 | Cl | $(CH_2)_4$ | Allyl | $(CH_2)_3$ | Br | |
| 66 | Br | $(CH_2)_4$ | Allyl | $(CH_2)_2$ | Br | |
| 67 | Br | $(CH_2)_4$ | Allyl | $(CH_2)_3$ | Br | |

Die wachstumsregulierenden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,001 und 3 kg oder mehr, vorzugsweise jedoch zwischen 0,01 und 1 kg Wirkstoff/ha.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten und Öldispersionen kommen Wasser oder Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon usw. in Betracht.

Wässrige Anwendungsformen können aus den Verbindungen als solchen oder aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermitel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäure, Alkylarylsulfonate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol, - Octylphenol, - Nonylphenol, Alkylphenol-polyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxy-

äthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Silikate, Talkum, Kaolin, Ton, Kalk, Talkum, Löss, Ton, Dolomit, Diatomeenerde, Calcium- und Magnesiumsulfat, gemahlene Kunststoffe, Düngemittel, wie z.B. Ammoniumsulfat, Ammoniumnitrat, Harnstoffe und pflanzliche Produkte. Wie Baumrinden-, Holz- und Nusschalenmehl, Cellulosepulver.

Die Anwendung erfolgt in der Weise, dass man die Pflanzen, die Samen oder den Erdboden mit den Mitteln behandelt, beispielsweise spritzt, sprüht, stäubt, beizt oder imprägniert.

Die erfindungsgemässen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrösserung des Wirkungsspektrums. Bei einer Anzahl solcher Wachstumsregulatormischungen treten auch synergistische Effekte auf, d.h. die Wirksamkeit des Kombinationsproduktes ist grösser als die addierten Wirksamkeiten der Einzelkomponenten.

In den nachfolgenden Beispielen wird die Wirkung der erfindungsgemäss verwendbaren Stoffe als Pflanzenwachstumsregulatoren dargestellt, ohne die Möglichkeit weiterer Anwendungen als Wachstumsregulatoren auszuschliessen.

Die Zumischung dieser Mittel zu den erfindungsgemässen Wirkstoffen kann im Gewichtsverhältnis 1:10 bis 10:1 erfolgen. Sie können gegebenenfalls auch erst unmittelbar vor der Anwendung (Tankmischung) zugesetzt werden.

Beispiel 5

Wirkung zu Sonnenblumen

Im Gewächshaus wurden Sonnenblumen in Kunststoffschalen von 11,5 cm Durchmesser in Torfkultursubstrat, welches ausreichend mit Nährstoffen versorgt war, eingesät. Die Wirkstoffe wurden in unterschiedlichen Aufwandmengen auf die Blätter gespritzt. Die Applikation erfolgte bei einer Wuchshöhe von 12 cm in üblicher Weise durch Spritzen.

Während der Wachstumszeit von 23 Tagen zeigten die behandelten Pflanzen gegenüber den unbehandelten Kontrollpflanzen ein deutlich geringeres Längenwachstum, was durch die abschliessenden Längenmessungen bestätigt werden konnte. Hierbei wurden von jeder Behandlungsreihe 14 Pflanzen gemessen.

Zum Vergleich wurde der bekannte Wirkstoff CCC (2-Chloräthyl-trimethyl-ammonium-chlorid) verwendet. Die Einzelmesswerte sind nachstehender Tabelle zu entnehmen.

Beeinflussung des Längenwachstums von Sonnenblumen bei Blattbehandlung

| Wirkstoff | Aufwandmenge kg/ha | Pflanzenhöhe cm | rel. |
|---|---|---|---|
| Kontrolle | – | 26,8 | 100 |
| CCC (bekannt) | 1,5 | 23,0 | 85,8 |
| 1 | 0,5 | 17,5 | 65,3 |
| | 1,0 | 17,0 | 63,4 |
| | 1,5 | 17,0 | 63,4 |

Beispiel 6

Wirkung zu Sojabohnen

In ähnlicher Weise wie zu Sonnenblumen wurde auch zu Sojabohnen ein Versuch in Kleinbehältern im Gewächshaus durchgeführt. Folgende Ergebnisse wurden nach einer Wachstumszeit von 36 Tagen ermittelt:

Beeinflussung des Längenwachstums von Sojabohnen im Gewächshausversuch mit Kleinbehältern bei Blattbehandlung

| Wirkstoff | Aufwandmenge kg/ha | Pflanzenhöhe cm | rel. |
|---|---|---|---|
| Kontrolle | – | 28,0 | 100 |
| CCC (bekannt) | 1,5 | 27,0 | 96,4 |
| | 6,0 | 26,5 | 94,6 |
| 1 | 1,5 | 10,5 | 37,5 |
| | 6,0 | 10,5 | 37,5 |

Beispiel 7

Wirkung zu Sojabohnen im Gefässversuch

Um den Wachstumsverlauf bei Sojabohnen über einen längeren Vegetationszeitraum beobachten zu können, wurde ein Versuch in grösseren Gefässen auf Lehm-Sandboden angesetzt. Die Düngung wurde mit 0,5 g N als Ammonnitrat und 0,5 g $P_2O_5$ als sek. Kaliumphosphat vorgenommen. Die Aufzucht der Pflanzen erfolgte in einer vollklimatisierten Gewächshauskammer unter optimalen Wachstumsbedingungen. Die Applikation der Wirkstoffe wurde bei einer Pflanzenhöhe von 20 cm durch Spritzen vorgenommen. Während der Versuchsdauer von 2 Monaten, in der die Kontrollreihen eine Wuchshöhe von ca. 60 cm erreichten, waren bei den behandelten Pflanzen Einkürzungen der Wuchshöhe bis zu 56% festzustellen, wobei die Pflanzen durch sehr kompakten Wuchs und stark dunkelgrüne Farbe aufielen. Die ermittelten Ergebnisse der Wuchshöhen sind in nachstehender Tabelle festgehalten.

Beeinflussung des Längenwachstums von Sojabohnen im Gefässversuch in der Klimakammer

| Wirkstoff | Aufwand-menge kg/ha | Pflanzenhöhe cm | rel. |
|---|---|---|---|
| Kontrolle | – | 58,6 | 100 |
| CCC (bekannt) | 0,5<br>1,0<br>1,5 | 60,0<br>59,0<br>58,0 | 102,4<br>100,7<br>99,0 |
| 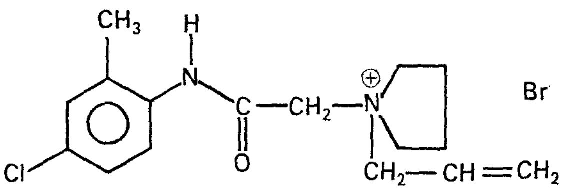<br>(DE-OS 26 57 728) | 0,5<br>1,0<br>1,5 | 41,0<br>30,0<br>29,0 | 70,0<br>51,2<br>49,5 |
| 1 | 0,5<br>1,0<br>1,5 | 34,5<br>28,5<br>25,5 | 58,9<br>48,6<br>43,5 |

Beispiel 8

Man vermischt 90 Gewichtsteile der Verbindung 1 mit 20 Gewichtsteilen Wasser und erhält eine Lösung, die zur Anwendung in Form kleinster Tropfen geeignet ist.

Beispiel 9

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

Beispiel 10

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel 11

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 12

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 13

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

Beispiel 14

40 Gewichtsteile des Wirkstoffs 1 werden mit 10 Teilen Natriumsalz eines Phenolsulfonsäureharnstoff-formaldehyd-Kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gewichtsprozent Wirkstoff enthält.

Beispiel 15

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäureharnstoff-formaldehyd-Kondensats und 68 Teilen eines paraffinischen Mineralöls innig vermischt. Man erhält eine stabile ölige Dispersion.

**Patentansprüche für die Vertragsstaaten: BE, CH, DE, FR, GB, IT, LI, LU, NL, SE**

1. Salz eines α-Aminoacetanilids der Formel I

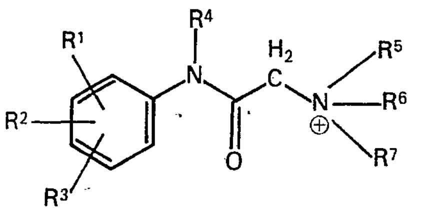

X I,

in der $R^1$, $R^2$, $R^3$ unabhängig voneinander Wasserstoff, einen Alkyl-, Cycloalkyl-, Aralkyl-, Alkoxyrest, wobei zwei benachbarte Reste einen heterocyclischen Ring mit 1 oder 2 Sauerstoffatomen bilden können, Halogen, Halogenalkyl, $NO_2$, Acyl, Alkoxyalkyl, Halogenalkoxy, Alkylthio, Halogenalkylthio, Aryl, Thiocyanato, Cyano, NHCOR′, NHCONR′R″, COOR′, CONR′R″, $SO_2R'$ oder $SO_2NR'R''$ bedeuten, wobei R′ und R″ unabhängig voneinander Wasserstoff, Alkyl, Cycloalkyl, Aryl oder Aralkyl bedeuten, $R^4$ einen Alkyl-, Aralkyl-, Alkenyl- oder Alkinylrest, wobei der Alkylrest mit der Orthoposition des aromatischen Ringes verknüpft sein kann, $R^5$, $R^6$, $R^7$ unabhängig voneinander gleiche oder verschiedene Reste aus der Gruppe der Alkyl-, Alkenyl-, Alkinyl-, Cyanoalkyl-, Halogenalkyl-, Hydroxyalkyl- oder Alkoxyalkylreste bedeuten, wobei zwei Alkylreste mit dem Stickstoffatom, dessen Substituenten sie sind, auch einen heterocyclischen Ring bilden können, der eine Doppelbindung enthalten kann, in dem ein Methylenrest durch ein Sauerstoffatom oder ein Schwefelatom ersetzt sein kann, und der durch Hydroxy-, Alkoxy-, Hydroxymethylen, Alkoxymethylen und Alkylgruppen substituiert sein kann und $X^\ominus$ das Anion einer Säure der Formel HBr, HCl, $HNO_3$, $CH_3OSO_3H$ oder $CF_3SO_3H$ ausser N-[2-(2,6-Dimethylanilino)-2-oxo]-ethyl-N-triethylammonium-bromid und N-[2-(2,6-Dimethylanilino)-2-oxo]-ethyl-N-triethylammonium-chlorid bedeutet.

2. N-[2-(4-Chlor-2-methyl-N-methyl-anilino)-2-oxo]-ethyl-N-allyl-pyrrolidiniumbromid.

3. Mittel zur Regulierung des Pflanzenwachstums, dadurch gekennzeichnet, dass sie ein Salz eines α-Aminoacetanilids gemäss Anspruch 1 enthalten.

4. Mittel zur Regulierung des Pflanzenwachstums gemäss Anspruch 3, enthaltend N-[2-(4-Chlor-2-methyl-N-methyl-anilino)-2-oxo]-ethyl-N-allyl-pyrrolidiniumbromid.

5. Mittel zur Regulierung des Pflanzenwachstums gemäss Anspruch 3, dadurch gekennzeichnet, dass sie einen festen oder flüssigen Trägerstoff enthalten und ein Salz eines α-Amino-acetanilids gemäss Anspruch 1.

6. Verfahren zur Regulierung des Pflanzenwachstums, dadurch gekennzeichnet, dass man die Pflanzen, ihren Samen oder den Boden behandelt mit einem Salz eines α-Aminoacetanilids gemäss Anspruch 1.

7. Verfahren zur Herstellung eines Salzes eines α-Aminoacetanilids gemäss Anspruch 1, in der $X^\ominus$ Chlorid oder Bromid bedeutet, dadurch gekennzeichnet, dass man ein α-Aminoacetanilid der Formel II

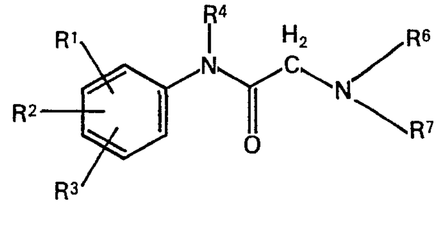

II

in der $R^1$ bis $R^4$, $R^6$ und $R^7$ die im Anspruch 1 angegebenen Bedeutungen haben, umsetzt mit einer Verbindung der Formel $R^5X$, in der $R^5$ die oben angegebene Bedeutung hat und X ein Chlor- oder Bromatom bedeutet.

**Patentansprüche für den Vertragsstaat: AT**

1. Mittel zur Regulierung des Pflanzenwachstums, enthaltend als Wirkstoff-Komponente ein Salz eines α-Aminoacetanilids der Formel I

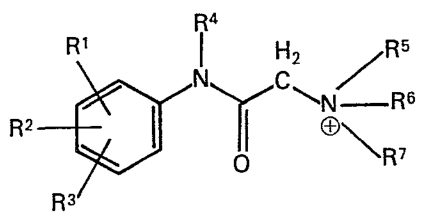

X I,

in der $R^1$, $R^2$, $R^3$ unabhängig voneinander Wasserstoff, einen Alkyl-, Cycloalkyl-, Aralkyl-, Alkoxyrest, wobei zwei benachbarte Reste einen heterocyclischen Ring mit 1 oder 2 Sauerstoffatomen bilden können, Halogen, Halogenalkyl, $NO_2$, Acyl, Alkoxyalkyl, Halogenalkoxy, Alkylthio, Halogenalkylthio, Aryl, Thiocyanato, Cyano, NHCOR′, NHCONR′R″, COOR′, CONR′R″, $SO_2R'$ oder $SO_2NR'R''$ bedeuten, wobei R′ und R″ unabhängig voneinander Wasserstoff, Alkyl, Cycloalkyl, Aryl oder Aralkyl bedeuten, $R^4$ einen Alkyl-, Aralkyl-, Alkenyl- oder Alkinylrest, wobei der Alkylrest mit der Orthoposition des aromatischen Ringes verknüpft sein kann, $R^5$, $R^6$, $R^7$ unabhängig voneinander gleiche oder verschiedene Reste aus der Gruppe der Alkyl-, Alkenyl-, Alkinyl-, Cyanoalkyl-, Halogenalkyl-, Hydroxyalkyl- oder Alkoxyalkylreste bedeuten, wobei zwei Alkylreste mit dem Stickstoffatom, dessen Substituenten sie sind, auch einen heterocyclischen Ring bilden können, der eine Doppelbindung enthalten kann, in dem ein Methylenrest durch ein Sauerstoffatom oder ein Schwefelatom ersetzt sein kann, und der durch Hydroxy-, Alkoxy-, Hydroxymethylen, Alkoxymethylen und Alkylgruppen substituiert sein kann und $X^\ominus$ das Anion einer Säure der Formel HBr, HCl, $HNO_3$, $CH_3OSO_3H$ oder $CF_3SO_3H$ ausser N-[2-(2,6-Dimethylanilino)-2-oxo]-ethyl-N-triethylammonium-bromid und N-[2-(2,6-Dimethylanilino)-2-oxo]-ethyl-N-triethylammonium-chlorid bedeutet.

2. Mittel zur Regulierung des Pflanzenwachstums gemäss Anspruch 1, enthaltend N-[2-(4-Chlor-2-methyl-N-methyl-anilino)-2-oxo]-ethyl-N-allyl-pyrrolidinium-bromid.

3. Mittel zur Regulierung des Pflanzenwachstums gemäss Anspruch 1, dadurch gekennzeichnet, dass sie einen festen oder flüssigen Trägerstoff enthalten und ein Salz eines α-Aminoacetanilids wie in Anspruch 1 definiert.

4. Verfahren zur Regulierung des Pflanzenwachstums, dadurch gekennzeichnet, dass man die Pflanzen, ihren Samen oder den Boden behandelt mit einem Salz eines α-Aminoacetanilids wie in Anspruch 1 definiert.

5. Verfahren zur Herstellung eines Salzes eines α-Aminoacetanilids gemäss Anspruch 1, in der $X^{\ominus}$ Chlorid oder Bromid bedeutet, dadurch gekennzeichnet, dass man ein α-Aminoacetanilid der Formel II

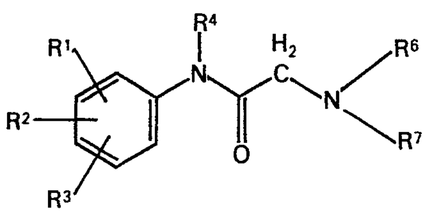 II

in der $R^1$ bis $R^4$, $R^6$ und $R^7$ die im Anspruch 1 angegebenen Bedeutungen haben, umsetzt mit einer Verbindung der Formel $R^5X$, in der $R^5$ die oben angegebene Bedeutung hat und X ein Chlor- oder Bromatom bedeutet.

**Revendications pour les Etats contractants: BE, CH, DE, FR, GB, IT, LI, LU, NL, SE**

1. Sel d'un α-aminoacétanilide de formule I

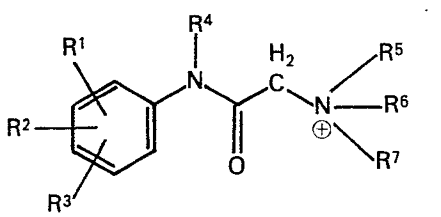 X I,

dans laquelle $R^1$, $R^2$, $R^3$ représentent indépendamment l'un de l'autre, l'hydrogène, un reste alkyle, cycloalkyle, aralkyle, alcoxy, deux restes voisins pouvant former un noyau hétérocyclique à 1 ou 2 atomes d'oxygène, un halogène, halogénoalkyle, $NO_2$, acyle, alcoxyalkyle, halogénoalcoxy, alkylthio, halogénoalkylthio, aryle, thiocyanato, cyano, NHCOR′, NHCONR′R″, COOR′, CONR′R″, $SO_2R'$ ou $SO_2NR'R''$, R′ et R″ représentant indépendamment l'un de l'autre, l'hydrogène, alkyle, cycloalkyle, aryle ou aralkyle, $R^4$ un reste alkyle, aralkyle, alcényle ou alcinyle, le reste alkyle pouvant être relié à la position ortho du noyau aromatique, $R^5$, $R^6$, $R^7$ représentent, indépendamment l'un de l'autre, des restes identiques ou différents appartenant au groupe des restes alkyle, alcényle, alcinyle, cyanoalkyle, halogénoalkyle, hydroxyalkyle ou alcoxyalkyle, deux restes alkyle avec l'atome d'azote, dont ils sont les substituants, pouvant former aussi une chaîne hétérocyclique, qui peut contenir une double liaison dans laquelle un reste méthyle peut être remplacé par un atome d'oxygène ou un atome de soufre et qui peut être substitué par des groupes hydroxy, alcoxy, hydroxyméthylène, alcoxyméthylène et alkyle et $X^{\ominus}$ l'anion d'un acide de formule HBr, HCl, $HNO_3$, $CH_3OSO_3H$ ou $CF_3SO_3H$, outre bromure de N-[2-(2,6-diméthylanilino)-2-oxo]-éthyl-N-triéthylammonium et chlorure de N-[2-(2,6-diméthylanilino)-2-oxo]-éthyl-N-triéthylammonium.

2. Bromure de N-[2-(4-chloro-2-méthyl-N-méthyl-anilino)-2-oxo]-éthyl-N-allyl-pyrrolidinium.

3. Moyen de régulation de la croissance des plantes, caractérisé par le fait qu'il contient un sel d'un α-aminoacétanilide selon la revendication 1.

4. Moyen de régulation de la croissance des plantes selon la revendication 3, contenant du bromure de N-[2-(4-chloro-2-méthyl-N-méthyl-anilino)-2-oxo]-éthyl-N-allyl-pyrrolidinium.

5. Moyen de régulation de la croissance des plantes selon la revendication 3, caractérisé par le fait qu'il contient un support solide ou liquide et un sel d'un α-aminoacétanilide selon la revendication 1.

6. Moyen de régulation de la croissance des plantes, caractérisé par le fait qu'on traite les plantes, leurs graines ou le sol avec un sel d'un α-aminoacétanilide selon la revendication 1.

7. Procédé de préparation d'un sel d'un α-aminoacétanilide selon la revendication 1, dans lequel $X^{\ominus}$ représente un chlorure ou un bromure, caractérisé par le fait qu'on fait réagir un α-aminoacétanilide de formule II

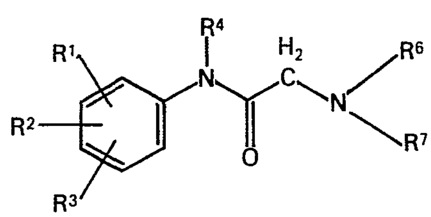 II

dans laquelle $R^1$ à $R^4$, $R^6$ et $R^7$ ont les significations données à la revendication 1, avec un composé de formule $R^5X$, dans laquelle $R^5$ a la même signification que ci-dessus et X représente un atome de chlore ou de brome.

**Revendications pour l'Etat contractant: AT**

1. Moyen de régulation de la croissance des plantes, contenant comme composant actif un sel d'un α-aminoacétanilide de formule I

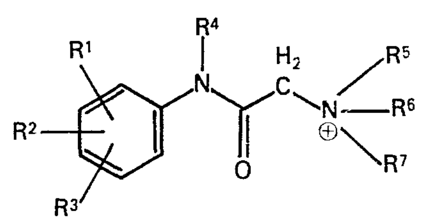 X I,

dans laquelle $R^1$, $R^2$, $R^3$ représentent indépendamment l'un de l'autre, l'hydrogène, un reste alkyle, cycloalkyle, aralkyle, alcoxy, deux restes voisins pouvant former un noyau hétérocyclique à 1 ou 2 atomes d'oxygène, un halogène, halogénoalkyle, $NO_2$, acyle, alcoxyalkyle, halogénoalcoxy, alkylthio, halogénoalkylthio, aryle, thiocyanato, cyano, NHCOR', NHCONR'R", COOR', CONR'R", $SO_2R'$ ou $SO_2NR'R''$, R' et R" représentant indépendamment l'un de l'autre, l'hydrogène, alkyle, cycloalkyle, aryle ou aralkyle, $R^4$ un reste alkyle, aralkyle, alcényle ou alcinyle, le reste alkyle pouvant être relié à la position ortho du noyau aromatique, $R^5$, $R^6$, $R^7$ représentent, indépendamment l'un de l'autre, des restes identiques ou différents appartenant au groupe des restes alkyle, alcényle, alcinyle, cyanoalkyle, halogénoalkyle, hydroxyalkyle ou alcoxyalkyle, deux restes alkyle avec l'atome d'azote, dont ils sont les substituants, pouvant former aussi une chaîne hétérocyclique, qui peut contenir une double liaison dans laquelle un reste méthyle peut être remplacé par un atome d'oxygène ou un atome de soufre et qui peut être substitué par des groupes hydroxy, alcoxy, hydroxyméthylène, alcoxyméthylène et alkyle et $X^{\ominus}$ l'anion d'un acide de formule HBr, HCl, $HNO_3$, $CH_3OSO_3H$ ou $CF_3SO_3H$, outre bromure de N-[2-(2,6-diméthylanilino)-2-oxo]-éthyl-N-triéthylammonium et chlorure de N-[2-(2,6-diméthylanilino)-2-oxo]-éthyl-N-triéthylammonium.

2. Moyen de régulation de la croissance des plantes selon la revendication 1, contenant du bromure de N-[2-(4-chloro-2-méthyl-N-méthylanilino)-2-oxo]-éthyl-N-allyl-pyrrolidinium.

3. Moyen de régulation de la croissance des plantes selon la revendication 1, caractérisé par le fait qu'il contient un support solide ou liquide et un sel d'un α-aminoacétanilide selon la revendication 1.

4. Moyen de régulation de la croissance des plantes, caractérisé par le fait qu'on traite les plantes, leurs graines ou le sol avec un sel d'un α-aminoacétanilide tel que défini à la revendication 1.

5. Procédé de préparation d'un seul d'un α-aminoacétanilide selon la revendication 1, dans lequel $X^{\ominus}$ représente un chlorure ou un bromure, caractérisé par le fait qu'on fait réagir un α-aminoacétanilide de formule II

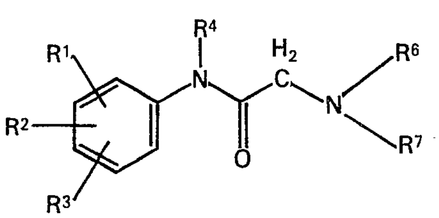

II

dans laquelle $R^1$ à $R^4$, $R^6$ et $R^7$ ont les significations données à la revendication 1, avec un composé de formule $R^5X$ dans laquelle $R^5$ a la même signification que ci-dessus et X représente un atome de chlore ou de brome.

**Claims for the Contracting States: BE, CH, DE, FR, GB, IT, LI, LU, NL, SE**

1. A salt of an α-aminoacetanilide of the formula I

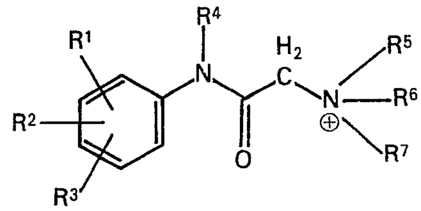

$X^{\ominus}$ I,

where $R^1$, $R^2$ and $R^3$, independently of one another, denote hydrogen, alkyl, cycloalkyl, aralkyl, alkoxy, it being possible for two adjacent radicals to form a heterocyclic ring with 1 or 2 oxygen atoms, $R^1$, $R^2$ and $R^3$ further denote halogen, haloalkyl, $NO_2$, acyl, alkoxyalkyl, haloalkoxy, alkylthio, haloalkylthio, aryl, thiocyanato, cyano, or NHCOR', NHCONR'R", COOR', CONR'R", $SO_2R'$, or $SO_2NR'R''$, R' and R", independently of one another, denoting hydrogen, alkyl, cycloalkyl, aryl or aralkyl, $R^4$ denotes alkyl, aralkyl, alkenyl or alkynyl, it being possible for the alkyl to be attached to the o-position of the aromatic ring, $R^5$, $R^6$ and $R^7$, independently of one another, denote identical or different radicals from the group of alkyl, alkenyl, alkynyl, cyanoalkyl, haloalkyl, hydroxyalkyl or alkoxyalkyl radicals, it being possible for 2 alkyls also to form, with the nitrogen atom whose substituents they are, a heterocyclic ring which may contain a double bond, and in which a methylene radical may be replaced by an oxygen or sulfur atom, and which may be substituted by hydroxy, alkoxy, hydroxymethylene, alkoxymethylene or alkyl, and $X^{\ominus}$ denotes the anion of an acid of the formula HBr, HCl, $HNO_3$, $CH_3OSO_3H$ or $CF_3SO_3H$ except N-[2-(2,6-dimethylanilino)-2-oxo]-ethyl-N-triethylammonium bromide and N-[2-(2,6-dimethylanilino)-2-oxo]-ethyl-N-triethylammonium chloride.

2. N-[2-(4-chloro-2-methyl-N-methylanilino)-2-oxo]-ethyl-N-allylpyrrolidinium bromide.

3. An agent for regulating plant growth, characterized in that it contains a salt of an α-aminoacetanilide as claimed in claim 1.

4. An agent for regulating plant growth, as claimed in claim 3, containing N-[2-(4-chloro-2-methyl-N-methylanilino)-2-oxo]-ethyl-N-allylpyrrolidinium bromide.

5. An agent for regulating plant growth, as claimed in claim 3, characterized in that it contains a solid or liquid carrier and a salt of an α-aminoacetanilide as claimed in claim 1.

6. A process for regulating plant growth, characterized in that the plants, their seeds or the soil are treated with a salt of an α-aminoacetanilide as claimed in claim 1.

7. A process for the manufacture of a salt of an α-aminoacetanilide as claimed in claim 1, in which $X^{\ominus}$ denotes chloride or bromide, characterized in that an α-aminoacetanilide of the formula II

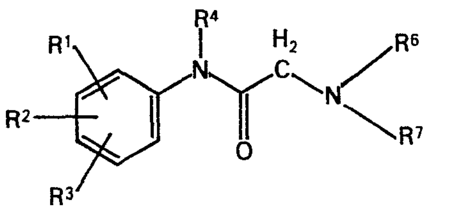

II

where $R^1$ to $R^4$, $R^6$ and $R^7$ have the meanings given in claim 1, is reacted with a compound of the formula $R^5X$, where $R^5$ has the above meanings and X denotes chlorine or bromine.

**Claims for the Contracting State: AT**

1. An agent for regulating plant growth, containing as active ingredient, a salt of an α-aminoacetanilide of the formula I

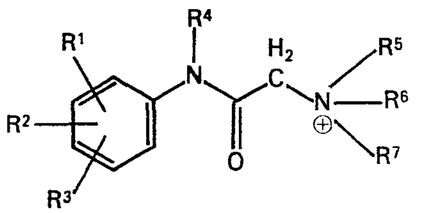

X I,

where $R^1$, $R^2$ and $R^3$, independently of one another, denote hydrogen, alkyl, cycloalkyl, aralkyl, alkoxy, it being possible for two adjacent radicals to form a heterocyclic ring with 1 or 2 oxygen atoms, $R^1$, $R^2$ and $R^3$ further denote halogen, haloalkyl, $NO_2$, acyl, alkoxyalkyl, haloalkoxy, alkylthio, haloalkylthio, aryl, thiocyanato, cyano, or NHCOR′, NHCONR′R″, COOR′, CONR′R″, $SO_2R'$, or $SO_2NR'R''$, R′ and R″, independently of one another, denoting hydrogen, alkyl, cycloalkyl, aryl or aralkyl, $R^4$ denotes alkyl, aralkyl, alkenyl or alkynyl, it being possible for the alkyl to be attached to the o-position of the aromatic ring, $R^5$, $R^6$ and $R^7$, independently of one another, denote identical or different radicals from the group of alkyl, alkenyl, alkynyl, cyanoalkyl, haloalkyl, hydroxyalkyl or alkoxyalkyl radicals, it being possible for 2 alkyls also to form, with the nitrogen atom whose substituents they are, a heterocyclic ring which may contain a double bond, and in which a methylene radical may be replaced by an oxygen or sulfur atom, and which may be substituted by hydroxy, alkoxy, hydroxymethylene, alkoxymethylene or alkyl, and $X^{\ominus}$ denotes the anion of an acid of the formula HBr, HCl, $HNO_3$, $CH_3OSO_3H$ or $CF_3SO_3H$ except N-[2-(2,6-dimethylanilino)-2-oxo]-ethyl-N-triethylammonium bromide and N-[2-(2,6-dimethylanilino)-2-oxo]-ethyl-N-triethylammonium chloride.

2. An agent for regulating plant growth, as claimed in claim 1, containing N-[2-(4-chloro-2-methyl-N-methylanilino)-2-oxo]-ethyl-N-allyl-pyrrolidinium bromide.

3. An agent for regulating plant growth, as claimed in claim 1, characterized in that it contains a solid or liquid carrier and a salt of an α-aminoacetanilide as defined in claim 1.

4. A process for regulating plant growth, characterized in that the plants, their seeds or the soil are treated with a salt of an α-aminoacetanilide as defined in claim 1.

5. A process for the manufacture of a salt of an α-aminoacetanilide as claimed in claim 1, in which $X^{\ominus}$ denotes chloride or bromide, characterized in that an α-aminoacetanilide of the formula II

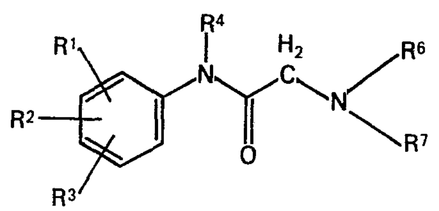

II

where $R^1$ to $R^4$, $R^6$ and $R^7$ have the meanings given in claim 1, is reacted with a compound of the formula $R^5X$, where $R^5$ has the above meanings and X denotes chlorine or bromine.